(19) Europäisches Patentamt
European Patent Office
Office européen des brevets

(11) Veröffentlichungsnummer: **0 006 540**
**B1**

(12) # EUROPÄISCHE PATENTSCHRIFT

(45) Veröffentlichungstag der Patentschrift: **07.10.81**

(51) Int. Cl.[3]: **C 07 D 215/08,** A 01 N 43/42

(21) Anmeldenummer: **79101941.7**

(22) Anmeldetag: **15.06.79**

(54) **N-Dichloracetyl-1,2,3,4-tetrahydro-chinaldin, Verfahren zu dessen Herstellung und dessen Verwendung zur Verhütung von Herbizidschäden an Kulturpflanzen sowie selektive herbizide Mittel auf Basis von N-Dichloracetyl-1,2,3,4-tetrahydro-chinaldin und herbizid wirksamen Acetaniliden oder Thiolcarbamaten.**

(30) Priorität: **28.06.78 DE 2828293**

(43) Veröffentlichungstag der Anmeldung: **09.01.80 Patentblatt 80/1**

(45) Bekanntmachung des Hinweises auf die Patenterteilung: **07.10.81 Patentblatt 81/40**

(84) Benannte Vertragsstaaten: **AT BE CH DE FR GB IT NL**

(56) Entgegenhaltungen:
**DE-A1-2 620 101**
**DE-A-2 218 097**
**FR-A-2 153 002**
**FR-A-2 368 476**
**GB-A-1 052 308**

(73) Patentinhaber: **BAYER AG, Zentralbereich Patente, Marken und Lizenzen, D-5090 Leverkusen 1, Bayerwerk (DE)**

(72) Erfinder: **Riebel, Hans-Jochem, Dr., Heimatstrasse 1, D-5418 Selters (DE)**
Erfinder: **Thomas, Rudolf, Dr., Wilkhausstrasse 129, D-5600 Wuppertal 2 (DE)**
Erfinder: **Eue, Ludwig, Dr., Paul-Klee-Strasse 36, D-5090 Leverkusen (DE)**
Erfinder: **Faust, Wilfried, Dr., Elfgenstrasse 16, D-5068 Odenthal 3 (DE)**
Erfinder: **Schmidt, Robert Rudolf, Dr., Hahnenweg 5, D-5000 Köln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 006 540 B1

# N-Dichloracetyl-1,2,3,4-tetrahydro-chinaldin, Verfahren zu dessen Herstellung und dessen Verwendung zur Verhütung von Herbizidschäden an Kulturpflanzen sowie selektive herbizide Mittel auf Basis von N-Dichloracetyl-1,2,3,4-tetrahydro-chinaldin und herbizid wirksamen Acetaniliden oder Thiolcarbamaten

Die vorliegende Erfindung betrifft das neue N-Dichloracetyl-1,2,3,4-tetrahydro-chinaldin, ein Verfahren zu dessen Herstellung sowie dessen Verwendung als Gegenmittel zur Verhütung von Herbizidschäden an Kulturpflanzen. Die Erfindung betrifft ferner herbizide Mittel auf Basis von N-Dichloracetyl-1,2,3,4-tetrahydro-chinaldin und herbizid wirksamen Acetaniliden oder Thiolcarbamaten.

Unter «Gegenmitteln» («Safener», «Antidote») sind im vorliegenden Zusammenhang Stoffe zu verstehen, welche befähigt sind, schädigende Wirkungen von Herbiziden auf Kulturpflanzen spezifisch zu antagonisieren, d.h. die Kulturpflanzen zu schützen, ohne dabei die Herbizidwirkung auf die zu bekämpfenden Unkräuter merklich zu beeinflussen.

Es ist bekannt, dass bestimmte Thiolcarbamate und Acetanilide beim Einsatz zur Unkrautbekämpfung in Mais und anderen Kulturen mehr oder weniger starke Schäden an den Kulturpflanzen hervorrufen. Weiterhin ist bekannt, dass Verbindungen wie z.B. N-Dichloracetyl-2-methyl-indolin und N-Dichloracetyl-cis,trans-decahydrochinolin geeignet sind, um Schädigungen durch Thiolcarbamate oder Acetanilide an Kulturpflanzen zu vermindern (vgl. DE–A 22 18 097). Die Wirksamkeit dieser Stoffe als Gegenmittel ist jedoch nicht immer ganz befriedigend.

Weiterhin sind bestimmte Heterocyclen mit antagonistischer Wirkung auf verschiedene Gruppen von Herbiziden bekannt (vgl. DE–A 2 620 101). In allen diesen Stoffen enthält der heterocyclische Ring jeweils ausser einem substituierten Stickstoffatom noch ein weiteres Heteroatom und der in Nachbarschaft zum Stickstoffatom vorhandene Rest kann nicht Methyl bedeuten.

Ferner ist bereits beschrieben worden, dass zahlreiche Amide von Chlor- und Brom-substituierten gesättigten aliphatischen Monocarbonsäuren mit 2 bis 4 Kohlenstoffatomen zum Schutz von Saatgut gegen Herbizidschäden eingesetzt werden können (vgl. FR–A 2 153 002). Unter anderem wird auch das N-Dichloracetyl-1,2,3,4-tetrahydroisochinolin erwähnt. Von diesem Stoff, in dem sich der Stickstoff im Heterocyclus nicht in Nachbarstellung zum anellierten Ring befindet, ist jedoch keine andere Applikationsart als die zur Saatgutbehandlung bekannt.

Es wurde jetzt das neue N-Dichloracetyl-1,2,3,4-tetrahydro-chinaldin der Formel

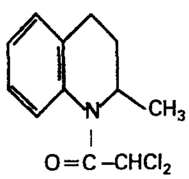

(I)

gefunden.

Weiterhin wurde gefunden, dass man das neue N-Dichloracetyl-1,2,3,4-tetrahydro-chinaldin der Formel (I) erhält, wenn man 1,2,3,4-Tetrahydrochinaldin der Formel

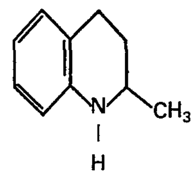

(II)

mit Dichloracetylchlorid gegebenenfalls in Gegenwart eines Säurebindemittels sowie gegebenenfalls in Gegenwart eines Verdünnungsmittels umsetzt.

Ausserdem wurde gefunden, dass N-Dichloracetyl-1,2,3,4-tetrahydro-chinaldin der Formel (I) hervorragend geeignet ist, um Kulturpflanzen vor Herbizidschädigungen durch Thiolcarbamate und/oder Acetanilide zu schützen.

Überraschenderweise werden Herbizidschädigungen durch Thiolcarbamate oder Acetanilide an Kulturpflanzen bei Mitverwendung des N-Dichloracetyl-1,2,3,4-tetrahydro-chinaldins besser unterdrückt als beim Einsatz der aus der DE–A 2 218 097 bekannten Verbindungen N-Dichloracetyl-2-methyl-indolin oder N-Dichloracetyl-cis, trans-decahydrochinolin, welches die chemisch ähnlichsten Stoffe gleicher Wirkungsart sind. Der erfindungsgemässe Stoff stellt somit eine wertvolle Bereicherung der Technik dar.

Die aufgefundene Wirksamkeit des N-Dichloracetyl-1,2,3,4-tetrahydro-chinaldins konnte auch im Hinblick auf die technische Lehre, welche der DE–A 2 620 101 zu entnehmen ist, nicht erwartet werden, denn die dort als Antidots beschriebenen Stoffe unterscheiden sind konstitutionell ganz erheblich von der erfindungsgemässen Verbindung.

Das aus der FR–A 2 153 002 als Antidot bekannte N-Dichloracetyl-1,2,3,4-tetrahydroisochinolin ist mit dem erfindungsgemässen Wirkstoff chemisch weniger verwandt als die oben genannten, in der DE–A 2 218 097 aufgeführten Komponenten. Im übrigen geht aus der FR–A 2 153 002 nur hervor, dass die dort erwähnten Stoffe bei einer Anwendung als Saatgutbehandlungsmittel ihre antagonistische Wirkung entfalten. Somit war auch nicht vorauszusehen, dass der erfindungsgemässe Wirkstoff auch anders als zur Saatgutbehandlung appliziert werden könnte.

Die Herstellung des N-Dichloracetyl-1,2,3,4-tetrahydro-chinaldins durch Umsetzung von 1,2,3,4-Tetrahydro-chinaldin mit Dichloracetylchlorid lässt sich durch das folgende Formelschema veranschaulichen:

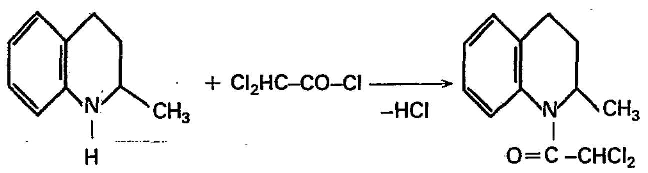


Das bei der Durchführung des erfindungsgemässen Verfahrens als Ausgangsstoff benötigte 1,2,3,4-Tetrahydrochinaldin der Formel (II) lässt sich durch Hydrierung von Chinaldin mit gasförmigem Wasserstoff in Gegenwart eines Katalysators, wie Raney-Nickel oder Ruthenium auf einem Trägerstoff, sowie gegebenenfalls in Gegenwart eines Verdünnungsmittels, wie Methanol oder Ethanol, bei Temperaturen zwischen 100 °C und 250 °C, vorzugsweise zwischen 150 °C und 200 °C herstellen. Der Wasserstoffdruck kann innerhalb eines grösseren Bereiches variiert werden. Im allgemeinen arbeitet man bei Drucken zwischen 100 und 220 bar, vorzugsweise zwischen 150 und 190 bar. Bevorzugt wird die Hydrierung von Chinaldin in Abwesenheit zusätzlicher Verdünnungsmittel unter Verwendung von Ruthenium auf Aluminiumoxid als Katalysator vorgenommen.

Bei der Durchführung des erfindungsgemässen Verfahrens kommen als Säurebindemittel alle üblichen Säureakzeptoren in Betracht. Hierzu gehören vorzugsweise Alkalihydroxide, wie Natriumhydroxid und Kaliumhydroxid, ferner Alkalicarbonate, wie Natriumcarbonat, Kaliumcarbonat und Natriumhydrogencarbonat, und weiterhin niedere tertiäre Amine, wie Triäthylamin, Dimethylbenzylamin, Pyridin und Diazabicyclooctan. Es kann jedoch auch im Überschuss eingesetztes 1,2,3,4-Tetrahydro-chinaldin der Formel (II) gleichzeitig als Säurebindemittel fungieren. In diesem Fall erübrigt sich die Zugabe eines zusätzlichen Säurebindemittels.

Als Verdünnungsmittel können bei der erfindungsgemässen Umsetzung Wasser sowie inerte organische Lösungsmittel verwendet werden. Hierzu gehören vorzugsweise Ketone, wie Diäthylketon, wie Methylisobutylketon; Nitrile, wie Propionitril und Acetonitril; Äther, wie Tetrahydrofuran oder Dioxan; aliphatische und aromatische Kohlenwasserstoffe, wie Petroläther, Benzol, Toluol oder Xylol, halogenierte Kohlenwasserstoffe, wie Methylenchlorid, Tetrachlorkohlenstoff, Chloroform oder Chlorbenzol; Ester, wie Essigester; und Formamide, wie insbesondere Dimethylformamid.

Die Reaktionstemperaturen können beim erfindungsgemässen Verfahren in einem grösseren Bereich variiert werden. Im allgemeinen arbeitet man zwischen etwa 0 und 60 °C, vorzugsweise zwischen 20 und 50 °C.

Bei der Durchführung des erfindungsgemässen Verfahrens setzt man auf 1 Mol 1,2,3,4-Tetrahydrochinaldin der Formel (II) vorzugsweise 1 Mol Dichloracetylchlorid und 1 Mol Säurebindemittel ein. Die Isolierung des Reaktionsproduktes erfolgt nach üblichen Methoden. Im allgemeinen verfährt man in der Weise, dass man das Reaktionsgemisch nach beendeter Umsetzung in Wasser giesst, das Gemisch mit einem in Wasser wenig löslichen organischen Solvens, wie z.B. Methylenchlorid ausschüttelt, die vereinigten organischen Phasen nacheinander mit verdünnter Salzsäure und Wasser wäscht, dann trocknet und eindampft und den verbleibenden Rückstand einer fraktionierten Destillation unterwirft.

Die praktische Herstellung des erfindungsgemässen N-Dichloracetyl-1,2,3,4-tetrahydro-chinaldins geht aus dem nachfolgenden Beispiel hervor.

Beispiel 1

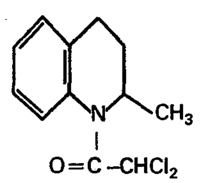

Eine Lösung von 29,4 g (0,2 Mol) 1,2,3,4-Tetrahydro-chinaldin in 200 ml Acetonitril wird bei Raumtemperatur unter Rühren mit 14,8 g (0,1 Mol) Dichloracetylchlorid versetzt. Danach wird 2 weitere Stunden bei 40 °C gerührt. Anschliessend lässt man abkühlen und gibt das Reaktionsgemisch in Wasser. Das entstehende Gemisch wird mehrfach mit Methylenchlorid extrahiert. Die vereinigten organischen Phasen werden nacheinander mit verdünnter Salzsäure und Wasser gewaschen, über Natriumsulfat getrocknet und unter vermindertem Druck eingeengt. Der verbleibende Rückstand wird fraktioniert destilliert. Man erhält auf diese Weise 20 g N-Dichloracetyl-1,2,3,4-tetrahydro-chinaldin.

Brechungsindex: $n_D^{20} = 1{,}5728$
Analyse: $C_{12}H_{13}ONCl_2$

| | | | | |
|---|---|---|---|---|
| Berechnet: | 55,83% C; | 5,08% H; | 27,47% Cl; | 5,43% Cl |
| Gefunden: | 55,6 % C; | 5,1 % H; | 27,3 % Cl; | 5,3 % Cl |

Das als Ausgangsmaterial benötigte 1,2,3,4-Tetrahydro-chinaldin der Formel

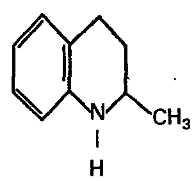

wird wie folgt erhalten:

143 g (1 Mol) Chinaldin werden mit 15 g eines Katalysatorgemisches, bestehend aus Ruthenium auf Aluminiumoxid, versetzt und 2 Stunden lang bei 195 °C unter einem Wasserstoffdruck von 170–190 bar hydriert. Danach wird das Reaktionsgemisch filtriert und einer fraktionierten Destillation unterworfen. Man erhält auf diese Weise 123 g an 1,2,3,4-Tetrahydro-chinaldin.

Siedepunkt: 75 °C bei 1 mm Hg

Das N-Dichloracetyl-1,2,3,4-tetrahydro-chinaldin der Formel (I) eignet sich, – wie bereits erwähnt –, zum Schutz von Kulturpflanzen vor Schädigungen durch herbizid wirksame Thiolcarbamate oder Acetanilide, ohne deren Unkrautwirkung merklich zu beeinflussen.

Vorzugsweise kann das N-Dichloracetyl-1,2,3,4-tetrahydro-chinaldin der Formel (I) als Gegenmittel bei herbizid wirksamen Thiolcarbamaten der Formel

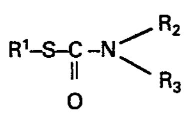

(III)

verwendet werden, in welcher

$R^1$ für $C_1$–$C_4$ Alkyl, Benzyl, Chlorbenzyl oder $C_1$–$C_4$ Alkoxybenzyl steht,

$R^2$ und $R^3$ unabhängig voneinander für Alkyl mit 2 bis 4 Kohlenstoffatomen oder für Cyclohexyl stehen und ausserdem

$R^2$ und $R^3$ gemeinsam mit dem angrenzenden Stickstoffatom für einen fünf- bis siebengliedrigen heterocyclischen Ring stehen.

Als Beispiele für Thiolcarbamate der Formel (III) seien im einzelnen genannt:

S-Äthyl-N,N-dipropylthiocarbamat
S-Äthyl-N,N-diisobutylthiocarbamat
S-Propyl-N-butyl-N-äthylthiocarbamat
S-Propyl-N,N-diisopropylthiocarbamat
S-Äthyl-N,N-diäthylthiocarbamat
S-Äthyl-N-äthyl-N-cyclohexylthiocarbamat
S-Äthyl-hexahydro-acepin-1-thiocarbamat
S-p-Methoxybenzyl-N,N-diäthylthiocarbamat
S-p-Chlorbenzyl-N,N-diäthylthiocarbamat
S-Benzyl-N,N-diäthylthiocarbamat
S-Benzyl-N,N-di-sek.-butylthiocarbamat
S-Propyl-N-äthyl-N-butylthiocarbamat

Die Thiolcarbamate der Formel (III) und deren herbizide Wirksamkeit sind bereits bekannt (vgl. US-Patentschriften 2 913 327, 3 037 853, 3 175 897, 3 185 720, 3 198 786 und 3 582 314).

Das N-Dichloracetyl-1,2,3,4-tetrahydro-chinaldin der Formel (I) kann ferner vorzugsweise als Gegenmittel verwendet werden bei herbizid wirksamen Acetaniliden der Formel

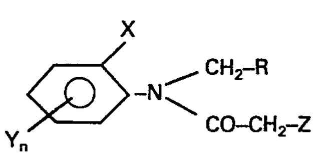

(IV)

in welcher

R für einen gegebenenfalls substituierten N-haltigen heterocyclischen Rest steht,

X und Y gleich oder verschieden sind und für Alkyl stehen,

Z für Halogen steht und

n für 0, 1 oder 2 steht, sowie bei deren herbizid wirksamen Säureadditionssalzen und Metallsalz-Komplexen.

Als Beispiele für Acetanilide der Formel (IV) seien im einzelnen genannt:

2-Methyl-6-äthyl-N-(pyrazol-1-yl-methyl)-chloracetanilid
2,6-Diäthyl-N-(pyrazol-1-yl-methyl)-chloracetanilid
2,6-diäthyl-N-(1,2,4-triazol-1-yl-methyl)-chloracetanilid
2,6-Dimethyl-N-(1,2,4-triazol-1-yl-methyl)-chloracetanilid
2-Methyl-N-(pyrazol-1-yl-methyl)-chloracetanilid
2,5-Dimethyl-N-(pyrazol-1-yl-methyl)-chloracetanilid
2,3-Dimethyl-N-(pyrazol-1-ylmethyl)-chloracetanilid
2-Methyl-6-äthyl-N-(pyrazol-1-yl-methyl)-chloracetanilid-hydrochlorid
2,6-Diäthyl-N-(pyrazol-1-yl-methyl)-chloracetanilid-hydrochlorid
2,6-Diäthyl-N[(3,5-dimethyl-pyrazol-1-yl)-methyl]-chloracetanilid
2,6-Diäthyl-N-[(3-chlor-1,2,4-triazolyl)-methyl]-chloracetanilid
2-Methyl-6-äthyl-N-[(3,5-dimethyl-pyrazol-1-yl)-methyl]-chloracetanilid
2-tert.-Butyl-N-(pyrazol-1-yl-methyl)-chloracetanilid
2-Methyl-6-äthyl-N-[(3-brom-5-methyl-pyrazolyl)-methyl]-chloracetanilid
2-Methyl-6-äthyl-N-[(3-chlor-1,2,4-triazolyl)-methyl]-chloracetanilid
2,6-Diäthyl-N-[(4-chlor-pyrazol-1-yl)-methyl]-chlor acetanilid

Weiterhin kann das N-Dichloracetyl-1,2,3,4-tetrahydro-chinaldin der Formel (I) vorzugsweise als Gegenmittel verwendet werden bei herbizid wirksamen Acetaniliden der Formel

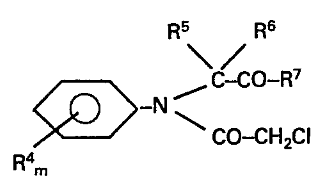

(V)

in welcher

$R^4$ für Alkyl, Halogen, Halogenalkyl, Alkylthio, Alkylsulfonyl, Aminosulfonyl, Cyano oder Nitro steht,

$R^5$ und $R^6$ gleich oder verschieden sind und für Wasserstoff, Alkyl, Halogen, Halogenalkyl oder gegebenenfalls substituiertes Phenyl stehen,

$R^7$ für Alkyl oder gegebenenfalls substituiertes Phenyl steht und

m für ganze Zahlen von 0 bis 5 steht.

Als Beispiele für Acetanilide der Formel (V) seien im einzelnen genannt:
2,6-Dimethyl-N-(benzol-methyl)-chloracetanilid
2,6-Dimethyl-N-(4-chlorbenzol-methyl)-chloracetanilid
2-Methyl-6-äthyl-N-(benzoyl-methyl)-chloracetanilid

Ausserdem kann das N-Dichloracetyl-1,2,3,4-tetrahydro-chinaldin der Formel (I) vorzugsweise als Gegenmittel verwendet werden bei herbizid wirksamen Acetaniliden der Formel

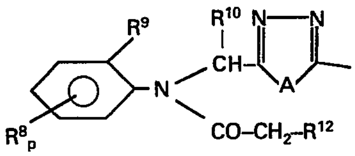
(VI)

in welcher
A für Sauerstoff, Schwefel oder die Gruppierung >$NR^{13}$ steht,
$R^{10}$ für Wasserstoff oder Alkyl steht,
$R^{11}$ für Wasserstoff, Alkyl, Halogenalkyl, Alkenyl, Alkinyl, Cycloalkyl, Halogen, gegebenenfalls substituiertes Aryl und Aralkyl oder die Gruppierungen $-OR^{14}$, $-SR^{14}$ und $NR^{13}R^{14}$ steht,
$R^{13}$ für Wasserstoff, Alkyl oder gegebenenfalls substituiertes Aryl steht,
$R^{14}$ für Wasserstoff, Alkyl, Halogenalkyl, Alkenyl, Alkinyl, Cycloalkyl oder gegebenenfalls substituiertes Aralkyl steht,
$R^8$ für Alkyl steht,
$R^9$ für Alkyl oder Halogen steht,
$R^{12}$ für Halogen steht und
p für die Zahlen 0, 1 oder 2 steht.

Als Beispiele für Acetanilide der Formel (VI) seien im einzelnen genannt:
2,6-Diäthyl-N-[(2-methyl-1,3,4-oxadiazol-5-yl)-methyl]-chloracetanilid
2,6-Dimethyl-N-[(2-methyl-1,3,4-oxadiazol-5-yl)-methyl]-chloracetanilid
2-Äthyl-6-methyl-N-[(2-methyl-1,3,4-oxadiazol-5-yl)-methyl]-chloracetanilid
2-tert.-Butyl-N-[(2-methyl-1,3,4-oxadiazol-5-yl)-methyl]-chloracetanilid

Darüber hinaus kann das N-Dichloracetyl-1,2,3,4-tetrahydro-chinaldin der Formel (I) vorzugsweise als Gegenmittel bei folgenden herbizid wirksamen Acetaniliden verwendet werden:
N-(2'-Methoxyäthyl)-2,6-dimethyl-chloracetanilid
N-(2'-Allyloxyäthyl)-2,6-dimethyl-chloracetanilid
N-(2'-n-Propyloxyäthyl)-2,6-dimethyl-chloracetanilid
N-(2'-Isopropyloxyäthyl)-2,6-dimethyl-chloracetanilid
N-(2'-Methoxyäthyl)-2-methyl-6-äthyl-chloracetanilid
N-(2'-Methoxyäthyl)-2,6-diäthyl-chloracetanilid
N-(2'-Äthoxyäthyl)-2-methyl-6-äthyl-chloracetanilid
N-(1'-Äthoxycarbonyl-äthyl)-2,6-dimethyl-chloracetanilid
N-(3'-Methoxy-propyl)-(2')-2-methyl-chloracetanilid
N-(3'-Methoxy-propyl-(2')-2,6-dimethyl-chloracetanilid
N-(3'-Methoxy-propyl-(2')-2-methyl-6-äthyl-chloracetanilid
N-(3'-Methoxy-propyl-(2')-2,6-diäthyl-chloracetanilid
N-(3'-Methoxy-propyl-(2')-2-äthyl-chloracetanilid
N-(2'-Äthoxyäthyl)-2,6-diäthyl-chloracetanilid
N-(2'-n-propoxyäthyl)-2-methyl-6-äthyl-chloracetanilid
N-(2'-n-Propoxyäthyl)-2,6-diäthyl-chloracetanilid
N-(2'-Isopropyloxyäthyl)-2-methyl-6-äthyl-chloracetanilid
N-Chloracetyl-2,6-dimethylanilino-essigsäure-äthyl- und methylester
β-(N-Chloracetyl-2,6-dimethylanilino)-propionsäure-methylester
α-(N-Chloracetyl-2-methyl-6-äthyl-anilino)-propionsäure-äthylester
N-(3'-Methoxy-propyl-(2')-2,3-dimethyl-chloracetanilid
N-(2'-Äthoxyäthyl)-2-methyl-6-chlor-chloracetanilid
N-(2'-Methoxyäthyl)-2-methyl-6-chlor-chloracetanilid
N-(2'-Methoxyäthyl)-2-methyl-6-methoxy-chloracetanilid

Die herbizid wirksamen Acetanilide der Formel (IV) sowie deren Säureadditionssalze und Metallsalz-Komplexe sind bereits bekannt (vgl. DE–A 2 648 008 und FR–A 2 379 525). Sie lassen sich herstellen, indem man
a) N-Halogenmethyl-halogenacetanilide der Formel

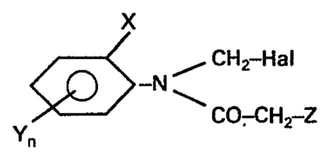
(VII)

in welcher
X, Y, Z und n die oben angegebene Bedeutung haben und Hal für Halogen, insbesondere Chlor oder Brom steht, mit Heterocyclen der Formel

R–M (VIII)

in welcher
R die oben angegebene Bedeutung hat und
M für Wasserstoff oder ein Alkalimetall steht, gegebenenfalls in Gegenwart eines Verdünnungsmittels und eines Säurebindemittels umsetzt und gegebenenfalls anschliessend eine Säure oder ein Metallsalz addiert.

In der Formel (VII) sind X und Y gleich oder verschieden und stehen vorzugsweise für geradkettiges oder verzweigtes Alkyl mit 1 bis 4 Kohlenstoffatomen. Z steht vorzugsweise für die Halogene Chlor oder Brom und der Index n hat die oben angegebene Bedeutung.

In der Formel (VIII) steht R vorzugsweise für die gegebenenfalls substituierten Azolylreste Pyrazol-1-yl, Imidazol-1-yl, 1,2,4-Triazol-1-yl; 1,2,3-Triazol-1-yl; 1,3,4-Triazol-1-yl und 1,2,3,4-Tetrazol-1-yl sowie für gegebenenfalls substituiertes Pyrrol-1-yl. Als Substituenten kommen vorzugsweise in Frage: Halogen, insbesondere Fluor, Chlor und Brom, sowie Alkyl mit 1 bis 4 Kohlenstoffatomen. M steht vorzugsweise für Wasserstoff und die Alkalimetalle Natrium und Kalium.

Zur Herstellung von Säureadditionssalzen der Verbindungen der Formel (IV) kommen alle Säuren in Frage, die zu physiologisch verträglichen Salzen führen. Hierzu gehören vorzugsweise die Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z.B. p-Toluolsulfonsäure und 1,5-Naphthalindisulfonsäure.

Zur Herstellung von Metallsalz-Komplexen der Verbindungen der Formel (IV) kommen vorzugsweise Salze von Metallen der II. bis IV. Haupt- und der I. und II. sowie IV. bis VIII. Nebengruppe in Frage, wobei Kupfer, Zink, Mangan, Magnesium, Zinn, Eisen und Nickel beispielhaft genannt seien. Als Anionen der Salze kommen solche in Betracht, die sich von Säuren ableiten, welche zu physiologisch verträglichen Salzen führen. Hierzu gehören vorzugsweise die Halogenwasserstoffsäuren, wie z.B. die Chlorwasserstoffsäure und die Bromwasserstoffsäure, ferner Phosphorsäure, Salpetersäure und Schwefelsäure.

Als besonders bevorzugte Acetanilide der Formel (IV) seien die folgenden Stoffe erwähnt:

Verbindung 3

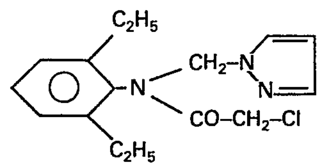

Schmelzpunkt: 67 °C

Tabelle 1

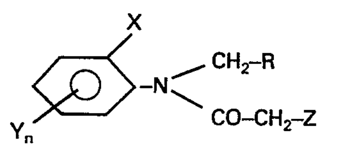

(IV)

| Verbindung Nr. | X | Yn | Z | R | Schmelzpunkt (°C) |
|---|---|---|---|---|---|
| 4 | $C_2H_5$ | 6–$C_2H_5$ | Cl | 1,2,4-Triazol-1-yl | 112 |
| 5 | i–$C_3H_7$ | 6–i–$C_3H_7$ | Cl | Pyrazol-1-yl | 134 |
| 6 | $CH_3$ | 6–$C_2H_5$ | Cl | 1,2,4-Triazol-1-yl | 92 |
| 7 | $CH_3$ | 6–$C_2H_5$ | Cl | Pyrazol-1-yl | 57 |
| 8 | $C_2H_5$ | 4,6–$(CH_3)_2$ | Cl | Pyrazol-1-yl | 82 |
| 9 | $CH_3$ | 4,6–$(CH_3)_2$ | Cl | Pyrazol-1-yl | 92 |
| 10 | $C_2H_5$ | 4–$CH_3$, 6–$C_2H_5$ | Cl | Pyrazol-1-yl | 78 |
| 11 | i–$C_3H_7$ | 6–i–$C_3H_7$ | Cl | 1,3,4-Triazol-1-yl | 196 |
| 12 | i–$C_3H_7$ | 6–i–$C_3H_7$ | Cl | 1,2,4-Triazol-1-yl | 138 |
| 13 | $C_2H_5$ | 6–$C_2H_5$ | Cl | Pyrrol-1-yl | Öl |
| 14 | i–$C_3H_7$ | – | Cl | 1,2,4-Triazol-1-yl | 118 |
| 15 | $CH_3$ | 6–$C_2H_5$ | Cl | 1,2,3,4-Tetrazol-1-yl | Öl |
| 16 | i–$C_3H_7$ | – | Cl | Pyrazol-1-yl | Öl |
| 17 | $C_2H_5$ | – | Cl | 1,2,4-Triazol-1-yl | 81 |
| 18 | $CH_3$ | 6–$CH_3$ | Cl | Pyrazol-1-yl | 82 |
| 19 | $CH_3$ | 6–$CH_3$ | Cl | 1,2,4-Triazol-1-yl | 110 |
| 20 | $CH_3$ | 5–$CH_3$ | Cl | 1,2,4-Triazol-1-yl | Öl |
| 21 | $CH_3$ | – | Cl | Pyrazol-1-yl | 56 |
| 22 | $CH_3$ | – | Cl | 1,2,4-Triazol-1-yl | 88 |
| 23 | $CH_3$ | 5–$CH_3$ | Cl | Pyrazol-1-yl | Öl |
| 24 | $CH_3$ | 3-$CH_3$ | Cl | 1,2,4-Triazol-1-yl | 114 |
| 25 | $CH_3$ | 3-$CH_3$ | Cl | Pyrazol-1-yl | 102 |
| 26 | $C_2H_5$ | 6–$CH_3$ | Cl | Pyrazol-1-yl (xHCl) | 87 |
| 27 | $C_2H_5$ | 6–$C_2H_5$ | Cl | Pyrazol-1-yl(xHCl) | 67 |
| 28 | $C_2H_5$ | 6–$C_2H_5$ | Cl | 3,5-Dimethyl-pyrazol-1-yl | 111 |
| 29 | $C_2H_5$ | 6–$C_2H_5$ | Cl | Brom-methyl-pyrazolyl | 145 |

Tabelle 1 (Fortsetzung)

| Verbindung Nr. | X | Yn | Z | R | Schmelzpunkt (°C) |
|---|---|---|---|---|---|
| 30 | $C_2H_5$ | 6–$C_2H_5$ | Cl | 3-Chlor-1,2,4-triazol-1-yl | 110 |
| 31 | $CH_3$ | 6–$C_2H_5$ | Cl | 3,5-Dimethyl-pyrazol-1-yl | 90 |
| 32 | $C_2H_5$ | 6–$C_2H_5$ | Cl | 3-Methyl-pyrazol-1-yl | 89 |
| 33 | $C_2H_5$ | 6–$CH_3$ | Cl | 3-Methyl-pyrazol-1-yl | 113 |
| 34 | $C(CH_3)_3$ | – | Cl | Pyrazol-1-yl | Öl |
| 35 | $C(CH_3)_3$ | – | Cl | 1,2,4-Triazol-1-yl | 118 |
| 36 | $C_2H_5$ | 6–$CH_3$ | Cl | Brom-methyl-pyrazolyl | 80 |
| 37 | $CH_3$ | 6–$C_2H_5$ | Cl | 4-Chlorpyrazol-1-yl | 91 |
| 38 | $CH_3$ | 6–$C_2H_5$ | Cl | 3-Chlor-1,2,4-triazol-1-yl | 121 |
| 39 | $C_2H_3$ | 6–$CH_3$ | Cl | 2,4,5-Trichlor-imidazol-1-yl | 158 |
| 40 | $C_2H_5$ | 6–$C_2H_5$ | Cl | 4-Chlorpyrazol-1-yl | 110 |
| 41 | $C_2H_5$ | 6–$C_2H_5$ | Cl | 1,2,3,4-Tetrazol-1-yl | 110 |
| 42 | $C_2H_5$ | 6–$C_2H_5$ | Br | Pyrazol-1-yl | 68 |
| 43 | $CH_3$ | 6–$C_2H_5$ | Br | Pyrazol-1-yl | 67 |
| 44 | $C_2H_5$ | 6–$C_2H_5$ | Cl | Imidazol-1-yl | Öl |
| 45 | $C_2H_5$ | 6–$C_2H_5$ | Br | 1,2,4-Triazol-1-yl | 90 |
| 46 | $CH_3$ | 6-$C_2H_5$ | Br | 1,2,4-Triazol-1-yl | 78 |

Die herbizid wirksamen Acetanilide der Formel (V) sind ebenfalls bereits bekannt (vgl. DE–A 27 26 253). Sie lassen sich herstellen, indem man N-Acylmethylaniline der Formel

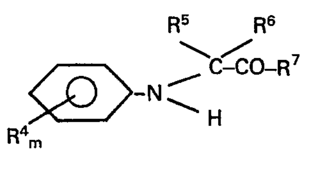

(IX)

in welcher

$R^4$, $R^5$, $R^6$, $R^7$ und m die bei Formel (V) angegebene Bedeutung haben, mit Chloracetylchlorid in Gegenwart eines Verdünnungsmittels umsetzt.

In der Formel (IX) steht $R^4$ vorzugsweise für geradkettiges oder verzweigtes Alkyl mit 1 bis 6, insbesondere 1 bis 4 Kohlenstoffatomen, Halogen, insbesondere Fluor, Chlor und Brom, Halogenalkyl mit bis zu 3 Kohlenstoff- und bis zu 5 gleichen oder verschiedenen Halogenatomen, wobei als Halogene insbesondere Fluor und Chlor stehen, beispielhaft sei Trifluormethyl genannt; ferner vorzugsweise für Alkylthio und Alkylsulfonyl mit 1 bis 4 Kohlenstoffatomen im Alkylteil sowie für Aminosulfonyl, Cyano und Nitro. $R^5$ und $R^6$ sind gleich oder verschieden und stehen vorzugsweise für Wasserstoff, geradkettiges oder verzweigtes Alkyl mit 1 bis 4 Kohlenstoffatomen, Halogen, insbesondere Fluor, Chlor und Brom, Halogenalkyl mit bis zu 3 Kohlenstoff- und bis zu 5 gleichen oder verschiedenen Halogenatomen, wobei als Halogene insbesondere Fluor und Chlor stehen sowie vorzugsweise für gegebenenfalls einfach oder mehrfach substituiertes Phenyl, wobei als Substituenten vorzugsweise die für $R^4$ genannten Reste in Frage kommen. $R^7$ steht vorzugsweise für geradkettiges oder verzweigtes Alkyl mit 1 bis 6, insbesondere 1 bis 4 Kohlenstoffatomen sowie für gegebenenfalls einfach oder mehrfach substituiertes Phenyl, wobei als Substituenten vorzugsweise die bei $R^4$ bereits genannten Reste sowie Phenyl und Phenoxy, die ebenfalls durch $R^4$ substituiert sein können, in Frage kommen.

Als besonders bevorzugte Acetanilide der Formel (V) seien die folgenden Stoffe erwähnt:

Verbindung 47

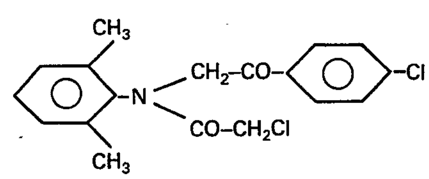

Schmelzpunkt: 128 °C.

Verbindung 48

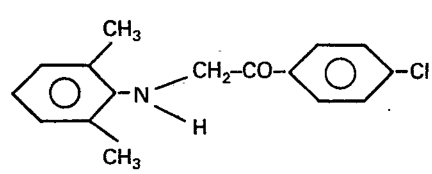

Schmelzpunkt: 86 °C.

Tabelle 2

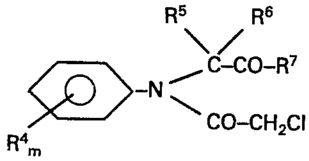

(V)

| Verbindung Nr. | $R^4{}_m$ | $R^5$ | $R^6$ | $R^7$ | Schmelzpunkt (°C) bzw. Brechungsindex |
|---|---|---|---|---|---|
| 49 | 2–$CH_3$ | H | H | 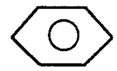 | 138 |
| 50 | 2–$CH_3$ | H | H | 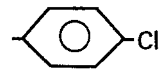 | 140 |
| 51 | 2,6–$(C_2H_5)_2$ | H | H | 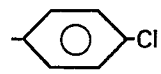 | 134 |
| 52 | 2,6–$(C_2H_5)_2$ | H | H | 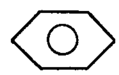 | 116 |
| 53 | 2–Cl | H | H | 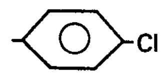 | 124 |
| 54 | 2,6–$(CH_3)_2$ | H | H | 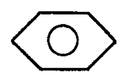 | 100 |
| 55 | 4–Cl | H | H | 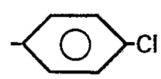 | 114 |
| 56 | 2,6–$(CH_3)_2$ | $CH_3$ | H | $CH_3$ | 104 |
| 57 | 2,6–$(i\text{–}C_3H_7)_2$ | H | H | 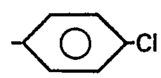 | 200 |
| 58 | 2,6–$(C_2H_5)_2$, 4-$CH_3$ | H | H | 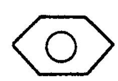 | 112 |
| 59 | 2,6–$(i\text{–}C_3H_7)_2$ | H | H | 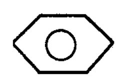 | 140 |
| 60 | 2,6–$(CH_3)_2$ | H | H | 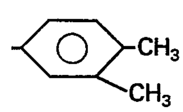 | 90 |
| 61 | 2–$C_2H_5$, 6-$CH_3$ | H | H | 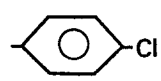 | 70 |
| 62 | 2,6–$(CH_3)_2$ | H | H | 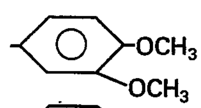 | 114 |
| 63 | 2–$C_2H_5$, 4,6–$(CH_3)_2$ | H | H | 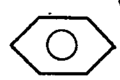 | $n_D^{20} = 1{,}5680$ |
| 64 | 2,6–$(CH_3)_2$ | H | H | 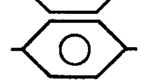 | 104 |
| 65 | 2,4,6–$(CH_3)_3$ | H | H | 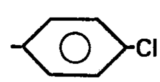 | 134 |

Tabelle 2 (Fortsetzung)

| Verbindung Nr. | $R^4{}_m$ | $R^5$ | $R^6$ | $R^7$ | Schmelzpunkt (°C) bzw. Brechungsindex |
|---|---|---|---|---|---|
| 66 | 2,4,6–$(CH_3)_3$ | H | H | 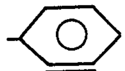 | $n_D^{20}=1{,}5610$ |
| 67 | 2,6–$(CH_3)_2$ | H | 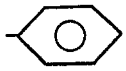 | 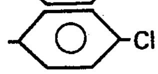 | 149 |
| 68 | 2,6–$(CH_3)_2$ | H | $CH_3$ | 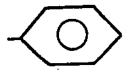 | 84 |
| 69 | 3,5–$(CF_3)_2$ | H | H | 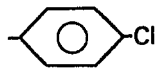 | |
| 70 | 2,6–$(CH_3)_2$ | H | H | 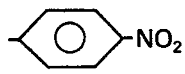 | |
| 71 | 2,6–$(CH_3)_2$ | H | H | 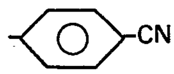 | |
| 72 | 2,6–$(CH_3)_2$ | H | H | 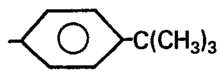 | |
| 73 | 2,6–$(CH_3)_2$, 4-$SO_2NH_2$ | H | H | 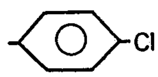 | |
| 74 | 2-Cl,6–$CH_3$ | H | H | 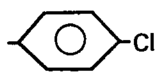 | |
| 75 | 2–$C_2H_5$,6–$CH_3$ | $CH_3$ | $CH_3$ | 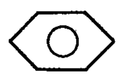 | |
| 76 | 2–$C_2H_5$,6–$CH_3$ | $CH_3$ | $CH_3$ | 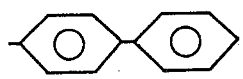 | |
| 77 | 2–$C_2H_5$,6–$CH_3$ | $CH_3$ | $CH_3$ | 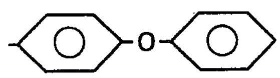 | |
| 78 | 2–$C_2H_5$,6–$CH_3$ | $CH_3$ | $CH_3$ | 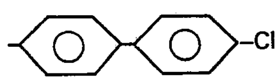 | |
| 79 | 2–$C_2H_5$,6–$CH_3$ | $CH_3$ | $CH_3$ | 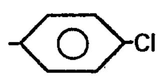 | |
| 80 | 2–$C_2H_5$,6–$CH_3$ | H | $CH_3$ | 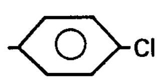 | |
| 81 | 2–$C_2H_5$,6–$CH_3$ | H | $CH_3$ | 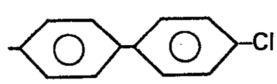 | |
| 82 | 2,6–$(CH_3)_2$ | H | 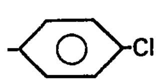 | 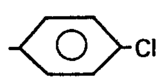 | |
| 83 | 2,6–$(CH_3)_2$ | H | 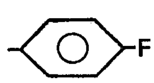 | 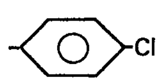 | |
| 84 | 2,6–$(CH_3)_2$ | H | 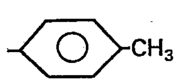 | 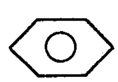 | |
| 85 | 2,6–$(CH_3)_2$ | H | 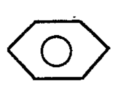 | 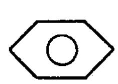 | |

Tabelle 2 (Fortsetzung)

| Verbindung Nr. | $R^4_m$ | $R^5$ | $R^6$ | $R^7$ |
|---|---|---|---|---|
| 86 | 2,6–$(CH_3)_2$ | H | 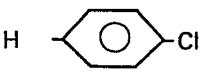 | 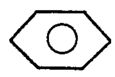 |
| 87 | 2,6–$(CH_3)_2$ | H | $CH_3$ | 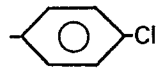 |

Die herbizid wirksamen Acetanilide der Formel (VI) sind ebenfalls bereits bekannt (vgl. DE–A 28 05 757). Sie lassen sich herstellen, indem man c) N-Azolylalkylaniline der Formel

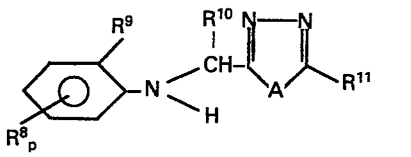

(X)

in welcher
$R^8$, $R^9$, $R^{10}$, $R^{11}$, A und p die oben angegebene Bedeutung haben, mit Halogenessigsäurechloriden bzw. -anhydriden der Formeln

$R^{12}–CH_2–CO–Cl$ (XIa)

bzw.

$(R^{12}–CH_2–CO)_2O$ (XIb)

in welchen
$R^{12}$ die oben angegebene Bedeutung hat, in Gegenwart eines Verdünnungsmittels und gegebenenfalls in Gegenwart eines Säurebindemittels umsetzt.

In der Formel (X) steht A vorzugsweise für Sauerstoff, Schwefel oder die Gruppierung $>NR^{13}$, worin $R^{13}$ vorzugsweise für Wasserstoff, geradkettiges oder verzweigtes Alkyl mit 1 bis 4 Kohlenstoffatomen sowie für Aryl mit 6 bis 10 Kohlenstoffatomen, insbesondere Phenyl, steht, wobei jeder dieser Arylreste substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoff- und bis zu 5 gleichen oder verschiedenen Halogenatomen, wobei als Halogene insbesondere Fluor und Chlor genannt seien. $R^{10}$ steht vorzugsweise für Wasserstoff oder Methyl. $R^{11}$ steht in der Formel (X) vorzugsweise für Wasserstoff, geradkettiges oder verzweigtes Alkyl mit 1 bis 4 Kohlenstoffatomen, Halogenalkyl mit bis zu 3 Kohlenstoff- und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Halogen, insbesondere Fluor, Chlor oder Brom. $R^{11}$ steht ferner vorzugsweise für Aryl mit 6 bis 10 Kohlenstoffatomen, insbesondere Phenyl, wobei jeder dieser Arylreste substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. $R^{11}$ steht ferner vorzugsweise für Aralkyl mit 6 bis 10 Kohlenstoffatomen im Arylteil und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei. Ausserdem steht $R^{11}$ für die Gruppierungen $–OR^{14}$, $–SR^{14}$ und $–NR^{13}R^{14}$, worin $R^{13}$ vorzugsweise für diejenigen Reste steht, die oben bereits vorzugsweise für diesen Rest genannt wurden, $R^{14}$ steht in diesen Gruppierungen für Wasserstoff, geradkettiges oder verzweigtes Alkyl mit 1 bis 4 Kohlenstoffatomen, Halogenalkyl mit bis zu 3 Kohlenstoffatomen und bis zu 5 gleichen oder verschiedenen Halogenatomen, wobei als Halogene insbesondere Fluor und Chlor stehen, beispielhaft sei Trifluormethyl genannt, ferner vorzugsweise für Alkenyl und Alkinyl mit 2 bis 4 Kohlenstoffatomen, Cycloalkyl mit 5 bis 7 Kohlenstoffatomen sowie für Aralkyl mit 6 bis 10 Kohlenstoffatom im Alkylteil, und 1 bis 4 Kohlenstoffatomen im Alkylteil, insbesondere für Benzyl, wobei jeder dieser Aralkylreste im Arylteil substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Cyano, Nitro und/oder Halogenalkyl mit bis zu 2 Kohlenstoffatomen und bis zu 5 gleichen oder verschiedenen Halogenatomen, wobei als Halogene insbesondere Fluor oder Chlor stehen und Trifluormethyl als Beispiel für Halogenalkyl speziell genannt sei.

In der Formel (X) steht $R^8$ vorzugsweise für geradkettiges oder verzweigtes Alkyl mit 1 bis 4 Kohlenstoffatomen. $R^9$ steht in der Formel (X) vorzugsweise für geradkettiges oder verzweigtes Alkyl mit 1 bis 4 Kohlenstoffatomen oder für die

Halogene Fluor, Chlor und Brom; der Index p steht für die Zahlen 0, 1 oder 2.

In den Formeln (XIa) und (XIb) steht $R^{12}$ vorzugsweise für Chlor, Brom und Jod.

Als besonders bevorzugte Acetanilide der Formel (VI) seien die folgenden Stoffe erwähnt:

Verbindung 88

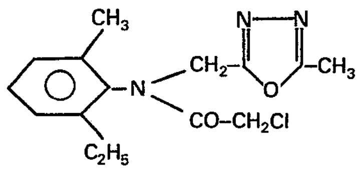

Schmelzpunkt: 67–70 °C

Verbindung 89

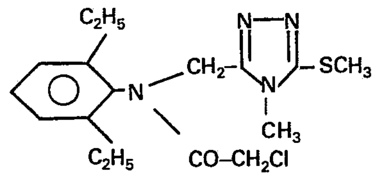

Schmelzpunkt: 121–123 °C

Tabelle 3

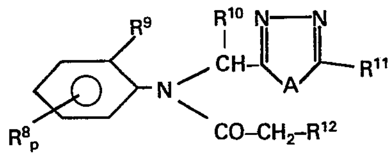

(VI)

| Verbindung Nr. | $R^{10}$ | $R^{11}$ | $R^9$ | $R^8_p$ | A | $R^{12}$ | Schmelzpunkt (°C) |
|---|---|---|---|---|---|---|---|
| 90 | H | $CH_3$ | $C_2H_3$ | 6-$C_2H_5$ | O | Cl | 79–82 |
| 91 | H | $CH_3$ | $CH_3$ | 6-$CH_3$ | O | Cl | 91–93 |
| 92 | H | $CH_3$ | $C(CH_3)_3$ | – | O | Cl | 102–04 |
| 93 | H | $-S-CH_2-CH=CH_2$ | $C_2H_3$ | 6-$C_2H_5$ | 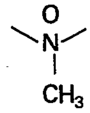 | Cl | 67–70 ° |
| 94 | H | 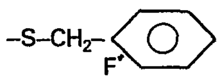 | $CH_3$ | 6-$C_3H_5$ | 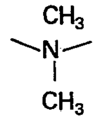 | Cl | 115–20 |
| 95 | H | $C_2H_5$ | $CH_3$ | 6-$C_2H_5$ | O | Cl | 57–59 |
| 96 | H | $C_2H_5$ | $C_2H_5$ | 6-$C_2H_5$ | O | Cl | 43–47 |
| 97 | H | i-$C_3H_7$ | $CH_3$ | 6-$C_2H_5$ | O | Cl | zähfl. Öl |
| 98 | H | $CH_3$ | $CH_3$ | 3-$CH_3$ | 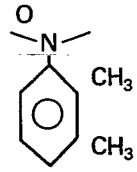 | Cl | glasartig erstarrt |
| 99 | H | $CH_3$ | $C_2H_5$ | 6-$C_2H_5$ | O | Br | 80 ° |
| 100 | H | $CH_3$ | $CH_3$ | 6-$C_2H_5$ | O | Br | 92–94 °C |
| 101 | H | $CH_3$ | i-$C_3H_7$ | 6-i-$C_3H_7$ | O | Cl | 135–37 ° |

Das erfindungsgemässe Gegenmittel, – das 1-Dichloracetyl-1,2,3,4-tetrahydro-chinaldin der Formel (I) –, eignet sich insbesondere zum Schutz von wichtigen Kulturpflanzen, wie Mais, Sojabohnen, Baumwolle, Zuckerrüben, Getreide, Reis und Zuckerrohr vor Herbizidschädigungen durch Thiolcarbamate und Acetanilide, insbesondere vor Schädigungen durch herbizide Wirkstoffe der Formeln (IV), (V) und (VI).

Die erfindungsgemässen Wirkstoffkombinationen zeigen eine sehr gute Wirkung gegen Unkräuter und Ungräser in zahlreichen Nutzpflanzenkulturen. Sie können daher zur selektiven Unkrautbekämpfung in zahlreichen Nutzpflanzenkulturen verwendet werden. – Unter Unkräutern im weitesten Sinne sind hierbei alle Pflanzen zu verstehen, die an Orten wachsen, wo sie unerwünscht sind.

Die erfindungsgemässen Wirkstoffkombinationen können zum Beispiel bei folgenden Pflanzen angewendet werden.

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cuburbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monocharia, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Insbesondere eignen sich die erfindungsgemässen Wirkstoffkombinationen zur selektiven Unkrautbekämpfung in Mais, Sojabohnen, Baumwolle, Zuckerrüben, Getreide, Reis und Zuckerrohr.

Das erfindungsgemässe Gegenmittel kann gegebenenfalls im Gemisch mit den herbiziden Wirkstoffen, bei denen es eingesetzt wird, in die üblichen Formulierungen übergeführt werden, wie Lösungen, Emulsionen, Spritzpulver, Suspensionen, Pulver, Stäubemittel, Schäume, Pasten, lösliche Pulver, Granulate, Suspensions-Emulsionskonzentrate, Saatgutpuder, Wirkstoff-imprägnierte Natur- und synthetische Stoffe, Feinstverkapselungen in polymeren Stoffen und in Hüllmassen für Saatgut.

Diese Formulierungen werden in bekannter Weise hergestellt, z.B. durch Vermischen des erfindungsgemässen Gegenmittels gegebenenfalls im Gemisch mit den herbiziden Wirkstoffen, bei denen es eingesetzt wird, mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene, oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; als feste Trägerstoffe natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehle, Kokosnussschalen, Maiskolben und Tabakstengel; als Emulgier- und/oder schaumerzeugende Mittel: nichtionogene und anionische Emulgatoren, wie Polyoxyäthylen-Fettsäure-Ester, Polyoxyäthylen-Fettalkohol-Äther, z.B. Alkylaryl-polyglykol-äther, Alkylsulfonate, Alkylsulfate, Arylsulfonate sowie Eiweisshydrolysate; als Dispergiermittel: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% an Gegenmittel bzw. an Gegenmittel und herbizidem Wirkstoff, vorzugsweise zwischen 0,5 und 90%.

Das erfindungsgemässe Gegenmittel kann als solches oder in seinen Formulierungen auch in Mischung mit herbiziden Wirkstoffen eingesetzt werden, wobei Fertigformulierung oder Tankmischung möglich ist. Auch eine Mischung mit anderen bekannten Wirkstoffen, wie Fungiziden, Insektiziden, Akariziden, Nematiziden, Schutzstoffen gegen Vogelfrass, Wuchsstoffen, Pflanzennährstoffen und Bodenstrukturverbesserungsmitteln ist möglich.

Das erfindungsgemässe Gegenmittel bzw. Gemische aus erfindungsgemässem Gegenmittel und herbizidem Wirkstoff können als solche, in Form ihrer Formulierungen oder den daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise, z.B. durch Giessen, Spritzen, Sprühen, Stäuben, Streuen, Trockenbeizen, Feuchtbeizen, Nassbeizen, Schlämmbeizen oder Inkrustieren.

Das erfindungsgemässe Gegenmittel kann nach den für derartige Antidote üblichen Methoden ausgebracht werden. So kann das erfindungsgemässe Gegenmittel entweder vor oder nach dem Herbizid ausgebracht oder zusammen mit dem Herbizid appliziert werden. Ferner können Kulturpflanzen durch Saatgutbehandlung mit dem Gegenmittel vor der Saat (Beizung) vor Schäden geschützt werden, wenn das Herbizid vor oder nach der Saat angewendet wird. Eine weitere Einsatzmöglichkeit besteht darin, dass man das Gegenmittel bei der Aussaat in die Saatfurche ausbringt. Wenn es sich bei den Pflanzen um Stecklinge handelt, so können diese vor der Auspflanzung mit dem Gegenmittel behandelt werden.

Beim Einsatz des erfindungsgemässen Gegenmittels kommen die ortsüblichen Aufwandmengen an den jeweiligen Herbiziden zur Anwendung.

Die Aufwandmengen an herbizidem Wirkstoff schwanken zwischen 0,5 und 5 kg/ha. Die Aufwandmenge an Gegenmittel ist unabhängig vom Herbizid und der Aufwandmenge des herbiziden Wirkstoffes. Im allgemeinen liegen die Aufwandmengen an erfindungsgemässem Gegenmittel bei Flächenbehandlung zwischen 0,1 und 5 kg/ha, vorzugsweise zwischen 0,2 und 4 kg/ha. Bei der Saatgutbehandlung liegen die Aufwandmengen an erfindungsgemässem Gegenmittel im allgemeinen zwischen 10 und 300 g pro Kilogramm Saatgut, vorzugsweise zwischen 25 und 200 g pro Kilogramm Saatgut.

In den erfindungsgemässen Wirkstoffkombinationen können die Gewichtsverhältnisse von Gegenmittel zu herbizidem Wirkstoff in relativ grossen Bereichen schwanken. Im allgemeinen entfallen auf 1 Gewichtsteil an herbizidem Wirkstoff der Formel (III), (IV), (V) oder (VI) 0,05 bis 1,0 Gewichtsteile, vorzugsweise 0,1 bis 0,5 Gewichtsteile an Gegenmittel der Formel (I).

Die gute Wirksamkeit des erfindungsgemässen Gegenmittels geht aus dem nachfolgenden Beispiel hervor.

Beispiel A
Pre-emergence-Test

Lösungsmittel: 5 Gew.-Teile Aceton
Emulgator: 1 Gew.-Teil Alkylarylpolyglykoläther

Zur Herstellung einer zweckmässigen Wirkstoffzubereitung vermischt man 1 Gew.-Teil herbiziden Wirkstoff bzw. Gegenmittel bzw. eines Gemisches aus Gegenmittel und herbizidem Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Böden ausgesät und nach 24 Stunden mit der Herbizid-Zubereitung bzw. mit der Gegenmittel-Zubereitung bzw. mit der Zubereitung aus Gegenmittel und herbizidem Wirkstoff begossen. Dabei hält man die Wassermenge pro Flächeneinheit zweckmässigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0% = keine Wirkung (wie unbehandelte Kontrolle)
100% = totale Vernichtung

Wirkstoffe, Aufwandmengen und Resultate gegen aus der nachfolgenden Tabelle hervor:

Tabelle A
Pre-emergence-Test

| Wirkstoff Herbizid | Wirkstoffaufwand an Herbizid kg/ha | Wirkstoff Gegenmittel | Wirkstoffaufwand an Gegenmittel kg/ha | Mais | Echinochloa | Amaranthus |
|---|---|---|---|---|---|---|
| – | – | 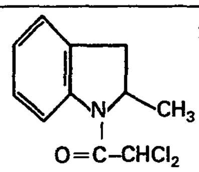 (bekannt) | 3 | 0 | 0 | 0 |
| – | – | 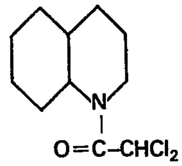 (bekannt) | 3 | 0 | 0 | 0 |
| – | – | 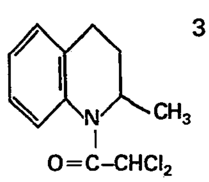 (erfindungsgemäss) | 3 | 0 | 0 | 0 |

Tabelle A (Fortsetzung)
Pre-emergence-Test

| Wirkstoff Herbizid | Wirkstoffaufwand an Herbizid kg/ha | Wirkstoff Gegen-mittel | Wirkstoffaufwand an Gegenmittel kg/ha | Mais | Echino-chloa | Ama-ranthus |
|---|---|---|---|---|---|---|
| 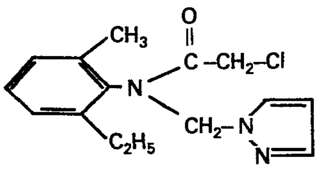 | 3 | – | – | 90 | 100 | 100 |
| 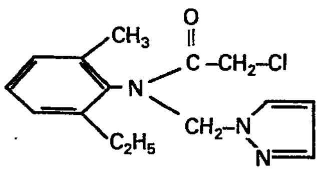 | 3 | 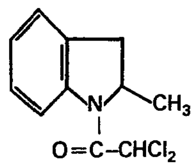 | 3 | 30 | 100 | 100 |
| 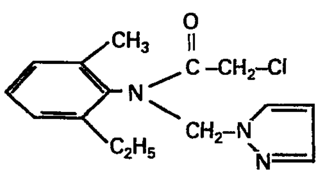 | 3 | 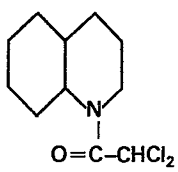 | 3 | 80 | 100 | 100 |
| 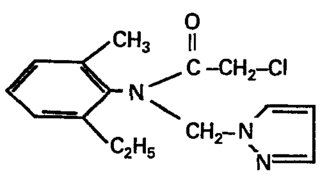 | 3 | 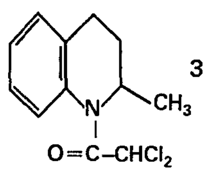 | 3 | 0 | 100 | 100 |

**Patentansprüche** für die Vertragsstaaten
BE, CH, DE, FR, GB, IT, NL

1) N-Dichloracetyl-1,2,3,4-tetrahydro-chinaldin der Formel

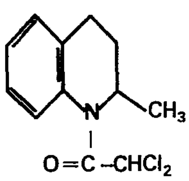

(I)

2) Verfahren zur Herstellung von N-Dichloracetyl-1,2,3,4-tetrahydro-chinaldin, dadurch gekennzeichnet, dass man 1,2,3,4-Tetrahydro-chinaldin der Formel

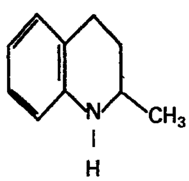

(II)

mit Dichloracetylchlorid gegebenenfalls in Gegenwart eines Säurebindemittels sowie gegebenenfalls in Gegenwart eines Verdünnungsmittels umsetzt.

3) Mittel zum Schutz von Kulturpflanzen vor Herbizidschädigungen durch Thiolcarbamate und/oder Acetanilide, gekennzeichnet durch einen Gehalt an N-Dichloracetyl-1,2,3,4-tetrahydro-chinaldin gemäss Anspruch 1.

4) Mittel gemäss Anspruch 3, gekennzeichnet durch einen Gehalt an
a) mindestens einem herbizid wirksamen Acetanilid der Formel

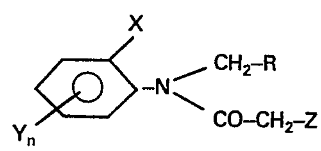

(IV)

in welcher
R für einen gegebenenfalls substituierten N-haltigen heterocyclischen Rest steht,
X und Y gleich oder verschieden sind und für Alkyl stehen,
Z für Halogen steht und
n für 0, 1 oder 2 steht,
bzw. einem herbizid wirksamen Säureadditionssalz oder Metallsalz-Komplex eines Acetanilides der Formel (IV),
oder einem herbizid wirksamen Acetanilid der Formel

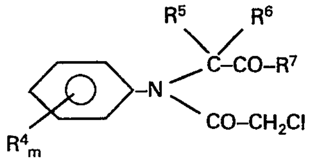

(V)

in welcher
$R^4$ für Alkyl, Halogen, Halogenalkyl, Alkylthio, Alkylsulfonyl, Aminosulfonyl, Cyano oder Nitro steht,
$R^5$ und $R^6$ gleich oder verschieden sind und für Wasserstoff, Alkyl, Halogen, Halogenalkyl oder gegebenenfalls substituiertes Phenyl stehen,
$R^7$ für Alkyl oder gegebenenfalls substituiertes Phenyl steht und
m für ganze Zahlen von 0 bis 5 steht,
oder einem herbizid wirksamen Acetanilid der Formel

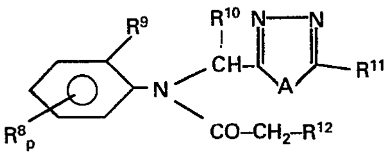

(VI)

in welcher
A für Sauerstoff, Schwefel oder die Gruppierung $>NR^{13}$ steht,
$R^{10}$ für Wasserstoff oder Alkyl steht,
$R^{11}$ für Wasserstoff, Alkyl, Halogenalkyl, Alkenyl, Alkinyl, Cycloalkyl, Halogen, gegebenenfalls substituiertes Aryl und Aralkyl oder die Gruppierungen $-OR^{14}$, $-SR^{14}$ und $NR^{13}R^{14}$ steht,
$R^{13}$ für Wasserstoff, Alkyl oder gegebenenfalls substituiertes Aryl steht,
$R^{14}$ für Wasserstoff, Alkyl, Halogenalkyl, Alkenyl, Alkinyl, Cycloalkyl oder gegebenenfalls substituiertes Aralkyl steht,
$R^8$ für Alkyl steht,
$R^9$ für Alkyl oder Halogen steht,
$R^{12}$ für Halogen steht und
p für die Zahlen 0, 1 oder 2 steht,
oder dem herbizid wirksamen Acetanilid der Formel

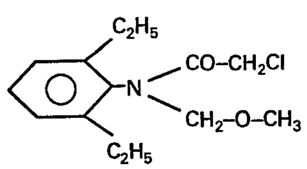

oder einem herbizid wirksamen Thiolcarbamat der Formel

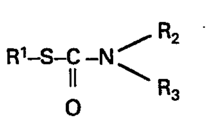

(III)

verwendet werden,
in welcher
$R^1$ für $C_1$–$C_4$ Alkyl, Benzyl, Chlorbenzyl oder $C_1$–$C_4$ Alkoxybenzyl steht,
$R^2$ und $R^3$ unabhängig voneinander für Alkyl mit 2 bis 4 Kohlenstoffatomen oder für Cyclohexyl stehen und ausserdem
$R^2$ und $R^3$ gemeinsam mit dem angrenzenden Stickstoffatom für einen fünf- bis siebengliedrigen heterocyclischen Ring stehen, und
b) dem als Gegenmittel wirksamen N-Dichloracetyl-1,2,3,4-tetrahydro-chinaldin der Formel (I).

5. Verwendung von N-Dichloracetyl-1,2,3,4-tetrahydro-chinaldin gemäss Anspruch 1 zum Schutz von Kulturpflanzen von Herbizidschädigungen durch Thiolcarbamate und/oder Acetanilide.

**Patentansprüche** für den Vertragsstaat AT

1) Verfahren zur Herstellung von N-Dichloracetyl-1,2,3,4-tetrahydro-chinaldin der Formel

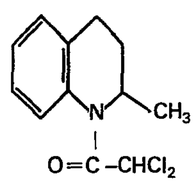

(I)

dadurch gekennzeichnet, dass man 1,2,3,4-Tetrahydro-chinaldin der Formel

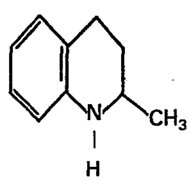

(II)

mit Dichloracetylchlorid gegebenenfalls in Gegenwart eines Säurebindemittels sowie gegebenenfalls in Gegenwart eines Verdünnungsmittels umsetzt.

2) Mittel zum Schutz von Kulturpflanzen vor Herbizidschädigungen durch Thiolcarbamate und/oder Acetanilide, gekennzeichnet durch einen Gehalt an N-Dichloracetyl-1,2,3,4-tetrahydrochinaldin der Formel (I).

3) Mittel gemäss Anspruch 2, gekennzeichnet durch einen Gehalt an
a) mindestens einem herbizid wirksamen Acetanilid der Formel

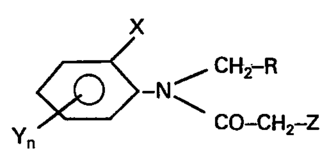

(IV)

in welcher
R für einen gegebenenfalls substituierten N-haltigen heterocyclischen Rest steht,
X und Y gleich oder verschieden sind und für Alkyl stehen,
Z für Halogen steht und
n für 0, 1 oder 2 steht,
bzw. einem herbizid wirksamen Säureadditionssalz oder Metallsalz-Komplex eines Acetanilides der Formel (IV),

oder einem herbizid wirksamen Acetanilid der Formel

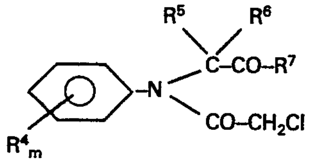

(V)

in welcher
$R^4$ für Alkyl, Halogen, Halogenalkyl, Alkylthio, Alkylsulfonyl, Aminosulfonyl, Cyano oder Nitro steht,
$R^5$ und $R^6$ gleich oder verschieden sind und für Wasserstoff, Alkyl, Halogen, Halogenalkyl oder gegebenenfalls substituiertes Phenyl stehen,
$R^7$ für Alkyl oder gegebenenfalls substituiertes Phenyl steht und
m für ganze Zahlen von 0 bis 5 steht,
oder einem herbizid wirksamen Acetanilid der Formel

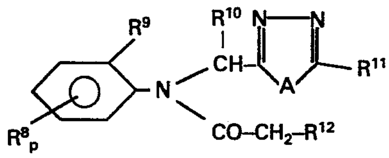

(VI)

in welcher
A für Sauerstoff, Schwefel oder die Gruppierung $>NR^{13}$ steht,
$R^{10}$ für Wasserstoff oder Alkyl steht,
$R^{11}$ für Wasserstoff, Alkyl, Halogenalkyl, Alkenyl, Alkinyl, Cycloalkyl, Halogen, gegebenenfalls substituiertes Aryl und Aralkyl oder die Gruppierungen $-OR^{14}$, $-SR^{14}$ und $NR^{13}R^{14}$ steht,
$R^{13}$ für Wasserstoff, Alkyl oder gegebenenfalls substituiertes Aryl steht,
$R^{14}$ für Wasserstoff, Alkyl, Halogenalkyl, Alkenyl, Alkinyl, Cycloalkyl oder gegebenenfalls substituiertes Aralkyl steht,
$R^8$ für Alkyl steht,
$R^9$ für Alkyl oder Halogen steht,
$R^{12}$ für Halogen steht und
p für die Zahlen 0, 1 oder 2 steht,
oder dem herbizid wirksamen Acetanilid der Formel

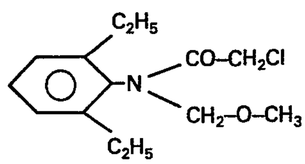

oder einem herbizid wirksamen Thiolcarbamat der Formel

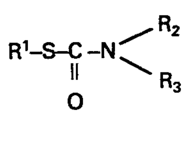

(III)

in welcher
$R^1$ für $C_1$–$C_4$ Alkyl, Benzyl, Chlorbenzyl oder $C_1$–$C_4$ Alkoxybenzyl steht,
$R^2$ und $R^3$ unabhängig voneinander für Alkyl mit 2 bis 4 Kohlenstoffatomen oder für Cyclohexyl stehen und ausserdem
$R^2$ und $R^3$ gemeinsam mit dem angrenzenden Stickstoffatom für eine fünf- bis siebengliedrigen heterocyclischen Ring stehen, und
b) dem als Gegenmittel wirksamen N-Dichloracetyl-1,2,3,4-tetrahydro-chinaldin der Formel (I).

4) Verwendung von N-Dichloracetyl-1,2,3,4-tetrahydro-chinaldin der Formel (I) zum Schutz von Kulturpflanzen vor Herbizidschädigungen durch Thiolcarbamate und/oder Acetanilide.

**Revendications** pour les Etats contractants BE, CH, DE, FR, GB, IT, NL

1. N-dichloroacétyl-1,2,3,4-tétrahydro-quinaldine de formule

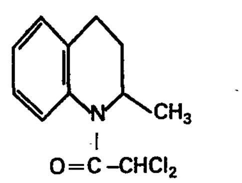

(I)

2. Procédé pour la préparation de la N-dichloroacétyl-1,2,3,4-tétrahydro-quinaldine, caractérisé en ce que l'on fait réagir la 1,2,3,4-tétrahydroquinaldine de formule

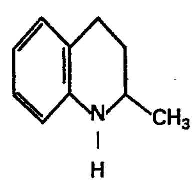

(II)

avec le chlorure de dichloroacétyle, éventuellement en présence d'un accepteur d'acide, ainsi éventuellement qu'en présence d'un agent diluant.

3. Agents pour la protection des cultures contre les dommages des herbicides par les thiolcarbamates et/ou acétanilides, caractérisés en ce qu'ils contiennent la N-dichloroacétyl-1,2,3,4-tétrahydro-quinaldine selon la revendication 1.

4. Agents selon la revendication 3, caractérisés en ce qu'ils contiennent
a) au moins un acétanilide efficace comme herbicide de formule

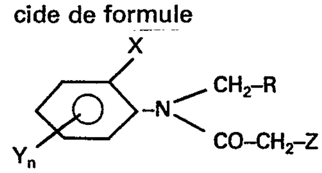

(IV)

dans laquelle
R représente un reste hétérocyclique azoté éventuellement substitué,
X et Y sont identiques ou différents et représentent des restes alkyles,
Z représente un halogène et
n représente 0, 1 ou 2,
ou bien un sel d'addition d'acide ou complexe de sel métallique efficace comme herbicide d'un

acétanilide de formule (IV) ou un acétanilide efficace comme herbicide de formule

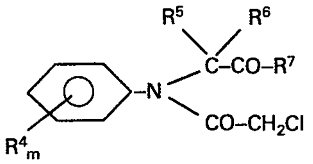

(V)

dans laquelle

$R^4$ représente un alkyle, un halogène, un halogènoalkyle, alkylthio, alkylsulfonyle, aminosulfonyle, cyano ou nitro,

$R^5$ et $R^6$ sont identiques ou différents et représentent l'hydrogène, un alkyle, un halogène, un halogénoalkyle ou un phényle éventuellement substitué,

$R^7$ représente un alkyle ou un phényle éventuellement substitué et

m représente des nombres entiers de 0 à 5,

ou un acétanilide efficace comme herbicide de formule

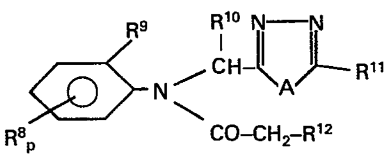

(VI)

dans laquelle

A représente l'oxygène, le soufre ou le groupe ment $>NR^{13}$,

$R^{10}$ représente l'hydrogène ou un alkyle,

$R^{11}$ représente l'hydrogène, un alkyle, un halogénoalkyle, un alcényle, un alcynyle, un cycloalkyle, un halogène, un aryle éventuellement substitué et un arylalkyle ou bien les groupements $-OR^{14}$, $-SR^{14}$ et $NR^{13}R^{14}$,

$R^{13}$ représente l'hydrogène, un alkyle ou un aryle éventuellement substitué,

$R^{14}$ représente l'hydrogène, un alkyle, un halogénoalkyle, un alcényle, un alcynyle, un cycloalkyle ou un arylalkyle éventuellement substitué,

$R^8$ représente un alkyle,

$R^9$ représente un alkyle ou un halogène,

$R^{12}$ représente un halogène et

p représente les nombres 0, 1 ou 2,

ou l'acétanilide efficace comme herbicide de formule

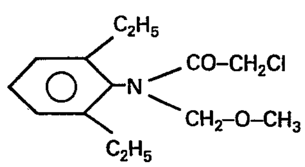

ou un thiolcarbamate efficace comme herbicide de formule

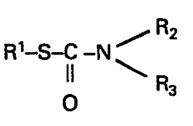

(III)

[«sont utilisés» ajoutés par erreur]

dans laquelle

$R^1$ représente un alkyle en $C_1$–$C_4$, un benzyle, un chlorobenzyle en un (alcoxy en $C_1$–$C_4$)-benzyle,

$R^2$ et $R^3$ représentent indépendamment l'un de l'autre un alkyle en $C_2$–$C_4$ ou un cyclohexyle et, en outre,

$R^2$ et $R^3$, pris ensemble avec l'atome d'azote voisin, représentent un noyau hétérocyclique ayant 5 à 7 chaînons; et

b) la N-dichloroacétyl-1,2,3,4-tétrahydro-quinaldine de formule (I) efficace comme antidote.

5. Utilisation de N-dichloroacétyl-1,2,3, 4-tétrahydro-quinaldine selon la revendication 1 pour la protection des cultures contre les dommages d'herbicides par les thiolcarbamates ou les acétanilides.

**Revendications** pour l'Etat contractant AT

1. Procédé pour la préparation de la N-dichloroacétyl-1,2,3,4,-tétrahydro-quinaldine de formule

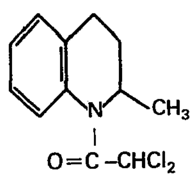

(I)

caractérisé en ce que l'on fait réagir la 1,2,3,4-tétrahydro-quinaldine de formule

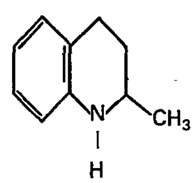

(II)

avec le chlorure de dichloroacétyle, éventuellement en présence d'un accepteur d'acide, ainsi éventuellement qu'en présence d'un agent diluant.

2. Agents pour la protection des cultures contre les dommages des herbicides par les thiolcarbamates et/ou acétanilides, caractérisés en ce qu'ils contiennent la N-dichloroacétyl-1,2,3,4-tétrahydro-quinaldine de formule (I).

3. Agents selon la revendication 2, caractérisés en ce qu'ils contiennent

a) au moins un acétanilide efficace comme herbicide de formule

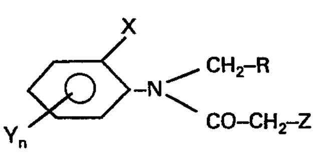

(IV)

dans laquelle

R représente un reste hétérocyclique azoté éventuellement substitué,

X et Y sont identiques ou différents et représentent des restes alkyles,

Z représente un halogène et

n représente 0, 1 ou 2,

ou bien un sel d'addition d'acide ou complexe de sel métallique efficace comme herbicide d'un acétanilide de formule (IV) ou un acétanilide efficace comme herbicide de formule

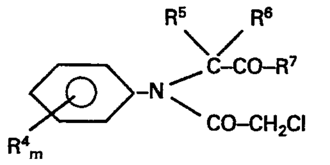
(V)

dans laquelle
$R^4$ représente un alkyle, un halogène, un halogénoalkyle, alkylthio, alkylsulfonyle, aminosulfonyle, cyano ou nitro,
$R^5$ et $R^6$ sont identiques ou différents et représentent l'hydrogène, un alkyle, un halogène, un halogénoalkyle ou un phényle éventuellement substitué,
$R^7$ représente un alkyle ou un phényle éventuellement substitué et
m représente des nombres entiers de 0 à 5,
ou un acétanilide efficace comme herbicide de formule

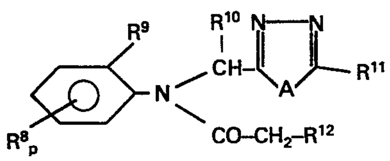
(VI)

dans laquelle
A représente l'oxygène, le soufre ou le groupement >$NR^13$,
$R^{10}$ représente l'hydrogène ou un alkyle,
$R^{11}$ représente l'hydrogène, un alkyle, un halogénoalkyle, un alcényle, un alcynyle, un cycloalkyle, un halogène, un aryle éventuellement substitué et un arylalkyle ou bien les groupements $-OR^{14}$, $-SR^{14}$ et $NR^{13}R^{14}$,
$R^{13}$ représente l'hydrogène, un alkyle ou un aryle éventuellement substitué,
$R^{14}$ représente l'hydrogène, un alkyle, un halogénoalkyle, un alcényle, un alcynyle, un cycloalkyle ou un arylalkyle éventuellement substitué,
$R^8$ représente un alkyle,
$R^9$ représente un alkyle ou un halogène,
$R^{12}$ représente un halogène et
p représente les nombres 0, 1 ou 2,
ou l'acétanilide efficace comme herbicide de formule

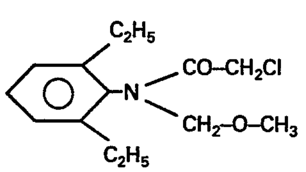

ou un thiol carbamate efficace comme herbicide de formule

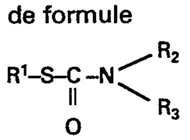
(III)

dans laquelle
$R^1$ représente un alkyle en $C_1$–$C_4$, un benzyle, un chlorobenzyle en un (alcoxy en $C_1$–$C_4$)-benzyle,
$R^2$ et $R^3$ représentent indépendamment l'un de l'autre un alkyle en $C_2$–$C_4$ ou cyclohexyl et, en outre,
$R^2$ et $R^3$, pris ensemble avec l'atome d'azote voisin, représentent un noyau hétérocyclique ayant 5 à 7 chaînons; et
b) la N-dichloroacétyl-1,2,3,4-tétrahydro-quinaldine de formule (I) efficace comme antidote.

4. Utilisation de N-dichloroacétyl-1,2,3,4-tetrahydro-quinaldine selon la revendication 1 pour la protection des cultures contre les dommages d'herbicides par les thiolcarbamates et/ou les acétanilides.

**Claims** for the contracting states:
BE, CH, DE, FR, GB, IT, NL

1. N-Dichloroacetyl-1,2,3,4-tetrahydro-quinaldine of the formula

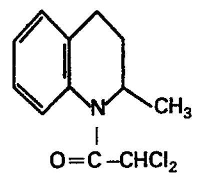
(I)

2. Process for the preparation of N-dichloroacetyl-1,2,3,4-tetrahydro-quinaldine, characterised in that 1,2,3,4-tetrahydro-quinaldine of the formula

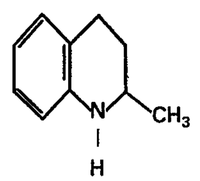
(II)

is reacted with dichloroacetyl chloride, if appropriate in the presence of an acid-binding agent and if appropriate in the presence of a diluent.

3. Agents for protecting crop plants against herbicidal damage by thiol-carbamates and/or acetanilides, characterised in that they contain N-dichloroacetyl-1,2,3,4-tetrahydro-quinaldine according to Claim 1.

4. Agents according to Claim 3, characterised in that they contain
a) at least one herbicidally active acetanilide of the formula

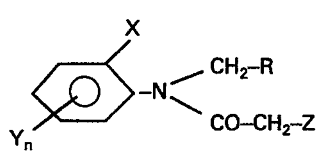
(IV)

in which
R represents an optionally substituted N-containing heterocyclic radical,
X and Y are identical or different and represent alkyl,

Z represents halogen and
n represents 0, 1 or 2, or a herbicidally active acid-addition salt or metal salt complex of an acetanilide of the formula (IV),
or a herbicidally active acetanilide of the formula

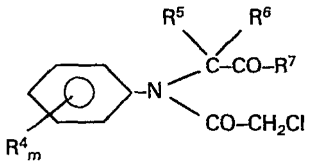
(V)

in which
$R^4$ represents alkyl, halogen, halogenoalkyl, alkylthio, alkylsulphonyl, aminosulphonyl, cyano or nitro,
$R^5$ and $R^6$ are identical or different and represent hydrogen, alkyl, halogen, halogenoalkyl or optionally substituted phenyl,
$R^7$ represents alkyl or optionally substituted phenyl and
m represents integers from 0 to 5,
or a herbicidally active acetanilide of the formula

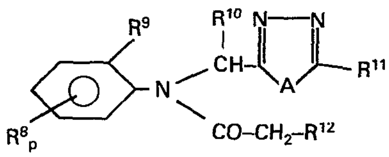
(VI)

in which
A represents oxygen, sulphur or the grouping $>NR^{13}$,
$R^{10}$ represents hydrogen or alkyl,
$R^{11}$ represents hydrogen, alkyl, halogenoalkyl, alkenyl, alkinyl, cycloalkyl, halogen, optionally substituted aryl and aralkyl or the groupings $-OR^{14}$, $-SR^{14}$ and $NR^{13}R^{14}$,
$R^{13}$ represents hydrogen, alkyl or optionally substituted aryl,
$R^{14}$ represents hydrogen, alkyl, halogenoalkyl, alkenyl, alkinyl, cycloalkyl or optionally substituted aralkyl,
$R^8$ represents alkyl,
$R^9$ represents alkyl or halogen,
$R^{12}$ represents halogen and
p represents the numbers 0, 1 or 2,
or the herbicidally active acetanilide of the formula

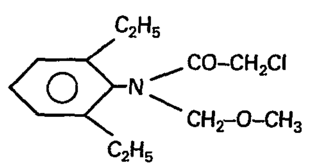

or a herbicidally active thiol-carbamate of the formula

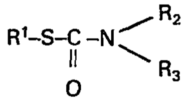
(III)

are used, in which
$R^1$ represents $C_1$–$C_4$ alkyl, benzyl, chlorobenzyl or $C_1$–$C_4$ alkoxybenzyl,
$R^2$ and $R^3$, independently or one another, represent alkyl with 2 to 4 carbon atoms or cyclohexyl and furthermore,
$R^2$ and $R^3$, together with the adjacent nitrogen atom, represent a five-membered to seven-membered heterocyclic ring, and
b) the antidotal N-dichloroacetyl-1,2,3,4-tetrahydro-quinaldine of the formula (I).

5. Use of N-dichloroacetyl-1,2,3,4-tetrahydro-quinaldine according to Claim 1 for protecting crop plants against herbicidal damage by thiolcarbamates and/or acetanilides.

**Claims** for the contracting state AT

1. Process for the preparation of N-dichloroacetyl-1,2,3,4-tetrahydro-quinaldine of the formula

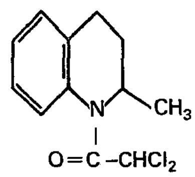
(I)

characterised in that 1,2,3,4-tetrahydro-quinaldine of the formula

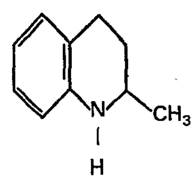
(II)

is reacted with dichloroacetyl chloride, if appropriate in the presence of an acid-binding agent and if appropriate in the presence of a diluent.

2. Agents for protecting crop plants against herbicidal damage by thiol-carbamates and/or acetanilides, characterised in that they contain N-dichloroacetyl-1,2,3,4-tetrahydro-quinaldine of the formula (I).

3. Agents according to Claim 2, characterised in that they contain
a) at least one herbicidally active acetanilide of the formula

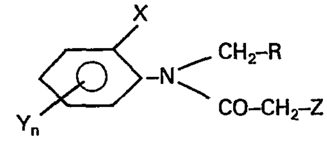
(IV)

in which
R represents an optionally substituted N-containing heterocyclic radical,
X and Y are identical or different and represent alkyl,
Z represents halogen and
n represents 0, 1 or 2,
or a herbicidally active acid-addition salt or metal

salt complex of an acetanilide of the formual (IV), or a herbicidally active acetanilide of the formula

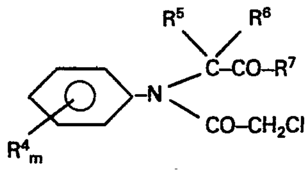

(V)

in which

$R^4$ represents alkyl, halogen, halogenoalkyl, alkylthio, alkylsulphonyl, aminosulphonyl, cyano or nitro,

$R^5$ and $R^6$ are identical or different and represent hydrogen, alkyl, halogen, halogenoalkyl or optionally substituted phenyl,

$R^7$ represents alkyl or optionally substituted phenyl and

m represents integers from 0 to 5,

or a herbicidally active acetanilide of the formula

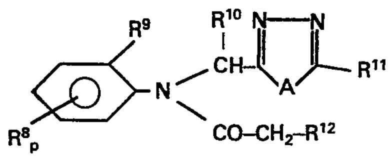

(VI)

in which

A represents oxygen, sulphur or the grouping $>NR^{13}$,

$R^{10}$ represents hydrogen or alkyl,

$R^{11}$ represents hydrogen, alkyl, halogenoalkyl, alkenyl, alkinyl, cycloalkyl, halogen, optionally substituted aryl and aralkyl or the groupings $-OR^{14}$, $-SR^{14}$ and $NR^{13}R^{14}$,

$R^{13}$ represents hydrogen, alkyl or optionally substituted aryl,

$R^{14}$ represents hydrogen, alkyl, halogenoalkyl, alkenyl, alkinyl, cycloalkyl or optionally substituted aralkyl,

$R^8$ represents alkyl,

$R^9$ represents alkyl or halogen,

$R^{12}$ represents halogen and

p represents the numbers 0, 1 or 2,

or the herbicidally active acetanilide of the formula

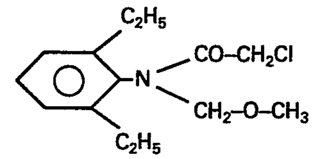

or a herbicidally active thiol-carbamate of the formula

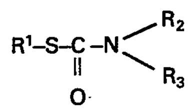

(III)

in which

$R^1$ represents $C_1$–$C_4$ alkyl, benzyl, chlorobenzyl or $C_1$–$C_4$ alkoxybenzyl,

$R^2$ and $R^3$, independently or one another, represent alkyl with 2 to 4 carbon atoms or cyclohexyl and furthermore

$R^2$ and $R^3$, together with the adjacent nitrogen atom, represent a five-membered to seven-membered heterocyclic ring, and

b) the antidotal N-dichloroacetyl-1,2,3,4-tetrahydro-quinaldine of the formula (I).

4. Use of N-dichloroacetyl-1,2,3,4-tetrahydroquinaldine of the formula (I) for protecting crop plants against herbicidal damage by thiol-carbamates and/or acetanilides.